# EUROPÄISCHE PATENTSCHRIFT

(11) **EP 1 150 968 B1**
(45) Veröffentlichungstag und Bekanntmachung des Hinweises auf die Patenterteilung: **07.01.2004**
(21) Anmeldenummer: 00907499.8
(22) Anmeldetag: 04.02.2000
(51) Int. Cl.: C07D 311/72, C07J 9/00

(54) **VERFAHREN ZUR HERSTELLUNG VON PHYTOSTERINEN**
METHOD FOR PRODUCING PHYTOSTEROLS
PROCEDE POUR LA PRODUCTION DE PHYTOSTEROLS

(30) Priorität: 13.02.1999 DE 19906551
(43) Veröffentlichungstag der Anmeldung: 07.11.2001
(73) Patentinhaber: Cognis Deutschland GmbH & Co. KG, 40589 Düsseldorf (DE)
(72) Erfinder: GUTSCHE, Bernhard, D-40724 Hilden (DE); SCHWARZER, Jörg, D-40723 Hilden (DE)
(86) Internationale Anmeldenummer: PCT/EP2000/000903
(87) Internationale Veröffentlichungsnummer: WO 2000/047570

(56) Entgegenhaltungen:
- DE-A- 4 228 476
- US-A- 3 335 154
- US-A- 4 422 974

## Beschreibung

### Gebiet der Erfindung

Die Erfindung befindet sich auf dem Gebiet der Lebensmittelzusatzstoffe und betrifft ein neues Verfahren zur Herstellung von Phytosterinen, die im wesentlichen frei von Citrostadienol sind.

### Stand der Technik

Phytosterine und deren Ester weisen hypocholesterinämische Eigenschaften auf, d.h. diese Stoffe sind in der Lage, den Cholesteringehalt im Blut zu reduzieren. Sie werden daher als Nahrungsmittelzusatzstoffe beispielsweise zur Herstellung von Margarine, Fritierölen, Wurst, Speiseeis und dergleichen eingesetzt. Die Gewinnung von Sterinen und anderen unverseifbaren Bestandteilen, wie z.B. Tocopherolen, aus Destillaten, die bei der Entsäuerung von pflanzlichen Ölen anfallen, ist in der Patentliteratur bereits verschiedentlich beschrieben worden. Stellvertretend seien hier die Druckschriften **EP-A2 0610742** (Hoffmann-LaRoche), **GB-A1 2145079** (Nisshin Oil Mills Japan) und **EP-A1 0333472** (Palm Oil Research and Development Board) genannt.

Aus dem Europäischen Patent **EP-B1 0656894** (Henkel) ist ein Verfahren zur Herstellung von Sterinen bekannt, bei dem man einen Rückstand aus der Methylesterdestillation, der im wesentlichen aus Glyceriden, Sterinen, Sterinestem und Tocopherolen besteht, in Gegenwart alkalischer Katalysatoren mit Methanol umestert. Nach Neutralisation des Katalysators, destillativer Abtrennung des überschüssigen Methanols und gegebenenfalls Auswaschen des Katalysators erfolgt die Kristallisation der Sterine durch Absenken der Reaktionstemperatur von etwa 65 auf 20 °C. Die dabei anfallenden Kristalle werden anschließend mit Methanol und Wasser gewaschen. Bei Einsatz von Rückständen aus der Herstellung von Methylestern auf Basis von Sonnenblumenöl werden jedoch Sterine erhalten, welche neben den Zielkomponenten, wie vor allem Campesterin, Capestanol, Stigmasterin, β-Sitosterin und β-Sitostanol auch wesentliche Mengen Citrostadienol enthalten, welches aus anwendungstechnischen Gründen eher unerwünscht ist. Aus der Deutschen Patentanmeldung **DE-A1 3226225** (Raisio) ist ein Verfahren bekannt, mit dessen Hilfe die Menge an Citrostadienol vermindert werden kann. Hierbei werden die festen Sterine zunächst in Heptan gelöst und dann nach Zugabe von Methanol erneut kristallisiert. Die resultierenden Produkte sind jedoch keineswegs frei von Citrostadienol, zudem sind die Ausbeuten unbefriedigend.

Demzufolge hat die Aufgabe der vorliegenden Erfindung darin bestanden, Phytosterine in hohen Ausbeuten zur Verfügung zu stellen, welche sich insbesondere dadurch auszeichnen, daß sie im wesentlichen frei von Citrostadienol sind.

### Beschreibung der Erfindung

Gegenstand der Erfindung ist ein Verfahren zur Herstellung von Phytosterinen durch alkalisch katalysierte Umesterung von Rückständen aus der Herstellung von Methylestern mit Methanol, Neutralisation des Katalysators und Abtrennung nicht umgesetzten Alkohols, welches sich dadurch auszeichnet, daß man die Umesterungsprodukte bei einer Temperatur, bei der sie in flüssigem Zustand vorliegen, in gesättigten Kohlenwasserstoffen mit 5 bis 10 Kohlenstoffatomen löst, die Phytosterine - gegebenenfalls unter Zugabe einer ausreichenden Menge wäßrigen Methanols - durch Absenken der Temperatur im Kohlenwasserstoff zur Kristallisation bringt sowie anschließend in an sich bekannter Weise durch Filtration, Wäsche und Trocknung abtrennt und aufreinigt.

Überraschenderweise wurde gefunden, daß sich bei Kristallisation der Sterine in Kohlenwasserstoffen durch Zugabe wirksamer Mengen wäßrigen Methanols Produkte erhalten lassen, welche Citrostadienolgehalte unterhalb von 0,5 und vorzugsweise unterhalb von 0,2 Gew.-% aufweisen und damit praktisch frei von dieser unerwünschten Komponente sind. Ein weiterer Vorteil besteht darin, daß im Gegensatz zu den Verfahren des Stands der Technik die Sterinausbeuten in der Kristallisation signifikant höher sind.

### Umesterung

Die Herstellung einer sterinreichen Fraktion durch Umesterung von Rückständen aus der Entsäuerung von Pflanzenölen und nachfolgende Aufarbeitung kann in der aus der EP-B1 0656894 bekannten Weise erfolgen. Als Ausgangsstoffe eignen sich Destillationsrückstände, die beispielsweise als sogenannte Dämpferkondensate bei der Herstellung von Fettsäuremethylestern auf Basis von Rapsöl oder insbesondere Sonnenblumenöl anfallen. Weiterhin geeignet ist Tallölpech, insbesondere Pech das aus Birkenrinde gewonnen wird. Soweit es die Herstellung der Sterinfraktionen betrifft, wird auf die oben genannte Druckschrift umfassend Bezug genommen.

### Kristallisation

Ein wesentliches Merkmal des neuen Verfahrens besteht darin, daß die aus der Umesterung erhältlichen Produkte bei einer solchen Temperatur in den Kohlenwasserstoffen gelöst werden, bei der sie noch flüssig sind. Vorzugsweise wird dies bei 60 bis 80 und insbesondere 65 bis 70°C der Fall sein. Als Lösemittel kommen flüssige Niedrigalkane, wie beispielsweise Pentan, Hexan, Heptan, Octan, Nonan und Decan in Frage. Eingeschlossen sind dabei sowohl die linearen Kohlenwasserstoffe sowie die sich denkgesetzlich davon ableitenden verzweigten Strukturisomeren und deren Gemische. Der Einsatz von Hexan, Heptan oder deren Gemische hat sich jedoch als besonders vorteilhaft erwiesen. Nach dem Lösen der Sterine wird die Temperatur so weit herabgesetzt, daß die reinen Sterine kristallisieren. Dabei hat es sich als vorteilhaft erwiesen, der Mischung eine wirksame Menge wäßrigen Methanols zuzusetzten. Üblicherweise werden zu diesem Zweck 1 bis 25 Gew.-%ige wäßrige Methanollösungen eingesetzt, wobei die Zusatzmenge dieser wäßrigen Methanollösungen bezogen auf die Kohlenwasserstoffe typischerweise etwa 1 bis 15 Gew.-% beträgt. Die Kristallisation beginnt zwar bereits bei einer Temperatur von etwa 30 °C, es hat sich jedoch als vorteilhaft erwiesen, die Temperatur auf etwa 15 bis 25 °C zu erniedrigen. Die anfallenden Phytosterine werden dann in an sich bekannter Weise abgetrennt und aufgereinigt, d.h. abfiltriert, seifenfrei gewaschen und bis zur Gewichtskonstanz getrocknet. Die resultierenden Produkte weisen einen Gehalt an Citrostadienol kleiner 0,5, vorzugsweise kleiner 0,2 Gew.-% auf.

### Beispiele

**Beispiel 1.** In einem 1-I-Dreihalskühler mit Rührer und Destillationsaufsatz wurden 200 g eines Destillationsrückstandes aus der Herstellung von Sonnenblumenfettsäuremethylester enthaltend u.a. 15 Gew.-% Glyceride und 28 Gew.-% freie oder gebundene Sterine zusammen mit 78 g Methanol vorgelegt. Die Mischung wurde mit 3,8 g 30 Gew.-%iger Natriummethylatlösung versetzt und 4 h bei 70°C gerührt. Anschließend wurde der alkalische Katalysator durch Zugabe von 4,2 g Citronensäure gelöst in 19 g Methanol neutralisiert, das nicht umgesetzte Methanol im Vakuum abdestilliert und der Rückstand bei 65°C mit Wasser seifenfrei gewaschen. Das Rohprodukt wurde mit einer Mischung aus 400 g Hexan, 26 g Methanol und 8 g Wasser versetzt und auf 20°C abgekühlt. Nach Abtrennen der Mutterlauge über einen Filter und Trocknen des Rückstands wurden 41 g Sterine erhalten, die frei von Citrostadienol waren.

**Beispiel 2.** Beispiel 1 wurde wiederholt, 100 g des Umesterungsprodukt jedoch mit einer Mischung aus 200 g Heptan, 13 g Methanol und 4 g Wasser versetzt und dann über einen Zeitraum von 4 h auf 20°C abgekühlt. Nach Filtration und Trocknung wurden 19,4 g Sterine mit einem Citrostadienol-Gehalt < 0,2 Gew.-% erhalten.

**Vergleichsbeispiel V1.** Beispiel 1 wurde wiederholt, das Umesterungsprodukt jedoch im Gewichtsverhältnis 1:1 mit Methanol versetzt. Bei Abkühlen auf 20°C schieden sich Kristalle ab, die abfiltriert, mit wäßrigem Methanol gewaschen und dann getrocknet wurden. Die resultierenden Sterine enthielten jedoch noch 4,7 Gew.-% Citrostadienol. 100 g dieses Produktes wurde bei 70°C in Heptan gelöst, anschließend mit 20 g Methanol versetzt und innerhalb von 4 h wieder auf 20°C abgekühlt. Nach Filtration und Trocknung wurden nur noch 75 g Sterine erhalten, die jedoch immer noch einen Citrostadienolgehalt von 4,2 Gew.-% aufwiesen.

## Patentansprüche

1. Verfahren zur Herstellung von Phytosterinen durch alkalisch katalysierte Umesterung von Rückständen aus der Herstellung von Methylestem mit Methanol, Neutralisation des Katalysators und Abtrennung nicht umgesetzten Alkohols, **dadurch gekennzeichnet, daß** man die Umesterungsprodukte bei einer Temperatur, bei der sie in flüssigem Zustand vorliegen, in gesättigten Kohlenwasserstoffen mit 5 bis 10 Kohlenstoffatomen löst, die Phytosterine durch Absenken der Temperatur im Kohlenwasserstoff zur Kristallisation bringt sowie anschließend in an sich bekannter Weise durch Filtration, Wäsche und Trocknung abtrennt und aufreinigt.

2. Verfahren nach Anspruch 1, **dadurch gekennzeichnet, daß** man Rückstände aus der Herstellung von Sonnenblumenfettsäuremethylestem oder Tallölpech einsetzt.

3. Verfahren nach den Ansprüchen 1 und/oder 2, **dadurch gekennzeichnet, daß** man die Umesterungsprodukte bei 60 bis 80 °C löst.

4. Verfahren nach mindestens einem der Ansprüche 1 bis 3, **dadurch gekennzeichnet, daß** man als Lösemittel Hexan, Heptan oder deren Gemische einsetzt.

5. Verfahren nach mindestens einem der Ansprüche 1 bis 4, **dadurch gekennzeichnet, daß** man während der Kristallisation eine wirksame Menge wäßrigen Methanols zugibt.

6. Verfahren nach mindestens einem der Ansprüche 1 bis 5, **dadurch gekennzeichnet, daß** man 1 bis 25 Gew.-%ige wäßrige Methanollösungen einsetzt.

7. Verfahren nach mindestens einem der Ansprüche 1 bis 6, **dadurch gekennzeichnet, daß** man die wäßrigen Methanollösungen bezogen auf die Kohlenwasserstoffe in Mengen von 1 bis 15 Gew.-% einsetzt.

8. Verfahren nach mindestens einem der Ansprüche 1 bis 7, **dadurch gekennzeichnet, daß** man Phytosterine mit einem Gehalt an Citrostadienol kleiner 0,5 Gew.-% herstellt.

## Claims

1. A process for the production of phytosterols by alkali-catalyzed transesterification of residues from the production of methyl esters with methanol, neutralization of the catalyst and removal of the unreacted alcohol, **characterized in that** the transesterification products are dissolved in saturated hydrocarbons containing 5 to 10 carbon atoms at a temperature at which they are present in liquid form, the phytosterols are crystallized in the hydrocarbon by lowering the temperature and are then removed and purified in known manner by filtration, washing and drying.

2. A process as claimed in claim 1, **characterized in that** residues from the production of sunflower oil fatty acid methyl esters or tall oil pitch are used.

3. A process as claimed in claims 1 and/or 2, **characterized in that** the transesterification products are dissolved at 60 to 80°C.

4. A process as claimed in at least one of claims 1 to 3, **characterized in that** hexane, heptane or mixtures thereof is/are used as the solvent.

5. A process as claimed in at least one of claims 1 to 4, **characterized in that** an effective quantity of aqueous methanol is added during crystallization.

6. A process as claimed in at least one of claims 1 to 5, **characterized in that** 1 to 25% by weight aqueous methanol solutions are used.

7. A process as claimed in at least one of claims 1 to 6, **characterized in that** the aqueous methanol solutions are used in quantities of 1 to 15% by weight, based on the hydrocarbons.

8. A process as claimed in at least one of claims 1 to 7, **characterized in that** phytosterols with a citrostadienol content of less than 0.5% by weight are produced.

## Revendications

1. Procédé de préparation de phytostérols par transestérification catalysée en milieu alcalin de résidus provenant de la préparation d'esters méthyliques avec le méthanol, neutralisation du catalyseur et séparation de l'alcool qui n'a pas réagi,
**caractérisé en ce qu'**
on dissout les produits de transestérification à une température à laquelle ils se présentent à l'état liquide, dans des hydrocarbures saturés ayant de 5 à 10 atomes de carbone, on amène les phytostérols à la cristallisation par abaissement de la température dans l'hydrocarbure ainsi qu'ensuite on sépare et purifie d'une manière connue en soi par filtration, lavage et séchage.

2. Procédé selon la revendication 1,
**caractérisé en ce qu'**
on met en oeuvre des résidus provenant de la préparation d'esters méthyliques d'acides gras de tournesol ou de brai d'huile de tallol.

3. Procédé selon les revendications 1 et/ou 2,
**caractérisé en ce qu'**
on dissout les produits de transestérification à 60 à 80 °C.

4. Procédé selon au moins une des revendications 1 à 3,
**caractérisé en ce qu'**
on met en oeuvre comme solvant de l'hexane, de l'heptane ou leurs mélanges.

5. Procédé selon au moins une des revendications 1 à 4,
**caractérisé en ce qu'**
on ajoute pendant la cristallisation une quantité efficace de méthanol aqueux.

6. Procédé selon au moins une des revendications 1 à 5,
**caractérisé en ce qu'**
on met en oeuvre de 1 à 25% en poids de solutions aqueuses de méthanol.

7. Procédé selon au moins une des revendications 1 à 6,
**caractérisé en ce qu'**
on met en oeuvre les solutions aqueuses de méthanol en quantité de 1 à 15% en poids rapporté aux hydrocarbures.

8. Procédé selon au moins une des revendications 1 à 7,
**caractérisé en ce qu'**
on prépare des phytostérols ayant une teneur en citrostadiénol inférieure à 0,5% en poids.
